# FASCICULE DE BREVET EUROPEEN

(11) **EP 0 946 921 B1**
(45) Date de publication et mention de la délivrance du brevet: **12.09.2001**
(21) Numéro de dépôt: 97952094.7
(22) Date de dépôt: 17.12.1997
(51) Int. Cl.: G06F 19/00

(54) **PROCEDE ET SYSTEME DE GESTION DE QUALITE DANS DES PROCESSUS THERAPEUTIQUES**
VERFAHREN UND SYSTEM ZUR QUALITÄTSVERWALTUNG IN THERAPEUTISCHEN PROZESSEN
METHOD AND SYSTEM FOR QUALITY MANAGEMENT IN THERAPEUTIC PROCESSES

(30) Priorité: 23.12.1996 FR 9615839
(43) Date de publication de la demande: 06.10.1999
(73) Titulaire: I.D.M. IMMUNO-DESIGNED MOLECULES, 75011 Paris (FR)
(72) Inventeur: THIBAUT, Eric, F-78760 Jouars-Pontchartrain (FR); ROMET-LEMONNE, Jean-Loup, F-75003 Paris (FR)
(74) Mandataire: Grosset-Fournier, Chantal Catherine
(86) Numéro de dépôt international: FR9702336
(87) Numéro de publication internationale: WO9828700

(56) Documents cités:
- WO-A-94/11838
- WO-A-94/27238
- US-A- 5 072 383
- US-A- 5 307 262
- VAN DYK ET AL: "commissioning and quality assurance of treatment planning computers" INTERNATIONAL JOURNAL OF RADIATION ONCOLOGY BIOLOGY PHYSICS, vol. 26, no. 2, 1993, pages 261-273, XP002064399

## Description

La présente invention concerne un procédé de gestion de qualité dans des processus thérapeutiques. Elle vise également un système pour sa mise en oeuvre.

Les nouveaux protocoles thérapeutiques intervenant au niveau des cellules et des gènes impliquent un haut niveau de fiabilité et de sécurité du fait de la complexité des opérations effectuées, du nombre d'étapes et d'intervenants impliqués dans le protocole. L'exigence de qualité est d'autant plus cruciale que ces nouvelles thérapies visent les briques élémentaires des être humains et font d'ailleurs l'objet d'une vigilance justifiée de la part des autorités sanitaires. Il est par ailleurs indispensable de garantir une traçabilité totales des prélèvements.

Des kits de thérapie cellulaire, par exemple le kit MAK™ produit par la société IDM, sont actuellement mis au point pour être fournis à des laboratoires chargés de réaliser des traitements sur des prélèvements cellulaires effectués sur des patients pour lesquels des cliniciens ont prescrit ce protocole thérapeutique. Les cellules ainsi traitées sont réinjectées sur ces patients.

Dans ces processus thérapeutiques, sont traités à la fois des éléments biologiques et des objets physiques: les poches de prélèvement associées chacune à un patient et des informations: des données associées à ces poches et indicatives notamment des intervenants et de l'état d'avancement du processus.

Les étapes de traitement des poches de cellules sont soumises à des procédures opératoires standard (SOP: "Standard Operating Procedure") totalement codifiées. Le respect de ces procédures garantit une qualité de traitement requise pour être agréé. Cependant, la complexité de ces procédures, la participation de plusieurs entités parties prenantes aux processus de traitement, la nécessité de rationaliser la gestion de processus thérapeutiques de plus en plus automatisés et le souci de vigilance exprimé par les autorités sanitaires, ont conduit au constat qu'il n'est pas possible de segmenter la responsabilité de la gestion de la qualité des processus thérapeutiques en ne comptant que sur chaque intervenant isolé dans le processus thérapeutique.

Le but de l'invention est de proposer un procédé de traitement d'informations utilisé pour la gestion de qualité qui garantisse une totale cohérence dans la quête de sécurité et de traçabilité tout au long des différentes étapes du processus thérapeutique.

Cet objectif est atteint avec un procédé et le système de traitement d'informations utilisé pour la gestion de qualité dans un processus thérapeutique selon les revendications 1 et 6, ce processus thérapeutique comportant des étapes de prélèvement de cellules sur un patient, un traitement spécifique de ces cellules selon un protocole de traitement spécifique, et une réinjection sur ce patient desdites cellules ainsi traitées.

Suivant l'invention, le procédé comprend:
- des étapes d'identification des entités impliquées dans le processus thérapeutique,
- des étapes de validation séquentielle et conditionnelle des étapes du processus thérapeutique, et
- des étapes de contrôle de qualité dans lesquelles des données acquises au cours desdites étapes de validation sont traitées pour fournir des informations sur la qualité de réalisation dudit processus thérapeutique, lesdites étapes d'identification, de validation et de contrôle étant réalisées pour chaque lot de prélèvement effectué sur un patient donné.

Ainsi, avec le procédé selon l'invention, il devient possible d'assurer le contrôle et le suivi de processus thérapeutiques complexes impliquant plusieurs entités et exigeant une traçabilité totale.

Un atout majeur du procédé de gestion de qualité selon l'invention réside en effet dans le fait qu'il est conçu autour de l'idée qu'à chaque patient est associé un lot, et qu'il s'agit de suivre systématiquement ce lot et les données qui lui sont attachées tout au long du processus. En particulier, les procédures d'exploitation standard (SOP) peuvent être suivies et validées, garantissant un haut niveau de traçabilité, de fiabilité, et de sécurité.

Lorsque le procédé de gestion de qualité selon l'invention est mis en oeuvre dans un processus thérapeutique impliquant plusieurs entités, éventuellement distantes, et est associé à une procédure opératoire standard de préparation comportant une succession d'étapes fonctionnelles, ce procédé comprend alors de préférence:
- des étapes de validation associées respectivement à chacune des étapes fonctionnelles, le passage d'une étape de validation à l'étape de validation suivante étant conditionné par des résultats d'un traitement de données collectées au cours de cette étape de validation, et
- une étape de traitement des informations et données collectées dans les différentes étapes de validation, pour émettre une certification finale d'une préparation réalisée selon la procédure opératoire standard et/ou une liste des irrégularités détectées au cours de cette préparation.

Dans un mode préféré de réalisation de l'invention, la validation de la certification finale est conditionnée à l'entrée d'un mot de passe de validation.

Le procédé de gestion de qualité selon l'invention est avantageusement mis en oeuvre sous la forme d'un logiciel installé sur un système de traitement d'informations. A chaque étape de validation est associée au moins une page-écran accessible sur des moyens de visualisation d'au moins un poste de travail connecté au système de traitement d'informations.

Chaque page-écran comprend un champ d'identification codée d'un patient auquel correspond le lot de prélèvement soumis à la procédure opératoire standard.

On peut avantageusement prévoir que la sortie de certaines des étapes du procédé soit conditionnée à l'impression de pages-écrans correspondant à ces étapes.

Lorsqu'il s'agit d'un procédé de gestion de qualité mis en oeuvre dans un laboratoire de préparation recevant d'au moins une entité exploitante des kits thérapeutiques, ce procédé comprend alors en outre des étapes pour contrôler le transfert de ces kits.

Lorsque le laboratoire de préparation est en relation avec un service de cytaphérèse, le procédé de gestion de qualité comprend en outre des étapes pour contrôler la réception de poches de cytaphérèse.

Suivant un autre aspect de l'invention, il est proposé un système de gestion de qualité pour la mise en oeuvre du procédé selon l'invention. Ce système de gestion de qualité peut en outre voir ses fonctions étendues à la gestion d'autres secteurs du laboratoire de préparation impliqués dans le processus thérapeutique. Par ailleurs, ce système est de préférence connecté à un réseau de communication, ouvert ou fermé, pour échanger des données avec d'autres entités impliquées dans un processus thérapeutique.

L'invention concerne un système de gestion de qualité permettant le paramétrage d'autres processus similaires de préparation protocolaire thérapeutique et ensuite leur contrôle et gestion au sein du même système.

D'autres particularités et avantages de l'invention apparaîtront encore dans la description ci-après. Aux dessins annexés donnés à titre d'exemples non limitatifs:
- la figure 1 illustre un système de gestion de qualité selon l'invention organisé autour d'un exploitant;
- la figure 2 illustre une organisation de gestion de qualité impliquant plusieurs processus thérapeutiques;
- la figure 3 représente les différentes étapes d'un processus thérapeutique auquel est associé un procédé de gestion de qualité selon l'invention;
- la figure 4 illustre les étapes essentielles du logiciel implémentant le procédé de gestion de qualité selon l'invention;
- la figure 5 représente schématiquement une page-écran offrant l'ensemble des étapes de gestion de qualité;
- la figure 6 représente schématiquement une page-écran correspondant à une étape de préparation d'un sérum autologue;
- la figure 7 représente schématiquement une page-écran correspondant à une étape de contrôle bactériologique;
- la figure 8 représente schématiquement une page-écran correspondant à une étape de saisie de résultats de tests de contrôle de qualité;
- la figure 9 représente schématiquement une page-écran correspondant à une étape d'analyse rétrospective;
- la figure 10 représente schématiquement une page-écran correspondant à une étape de certification finale;
- la figure 11 représente schématiquement une page-écran correspondant à une étape de listage des irrégularités; et
- la figure 12 représente schématiquement une page-écran correspondant à une étape de transfert du laboratoire de préparation vers le centre de traitement.

On va maintenant décrire un exemple de mise en oeuvre du procédé selon l'invention, dans le domaine de la thérapie cellulaire.

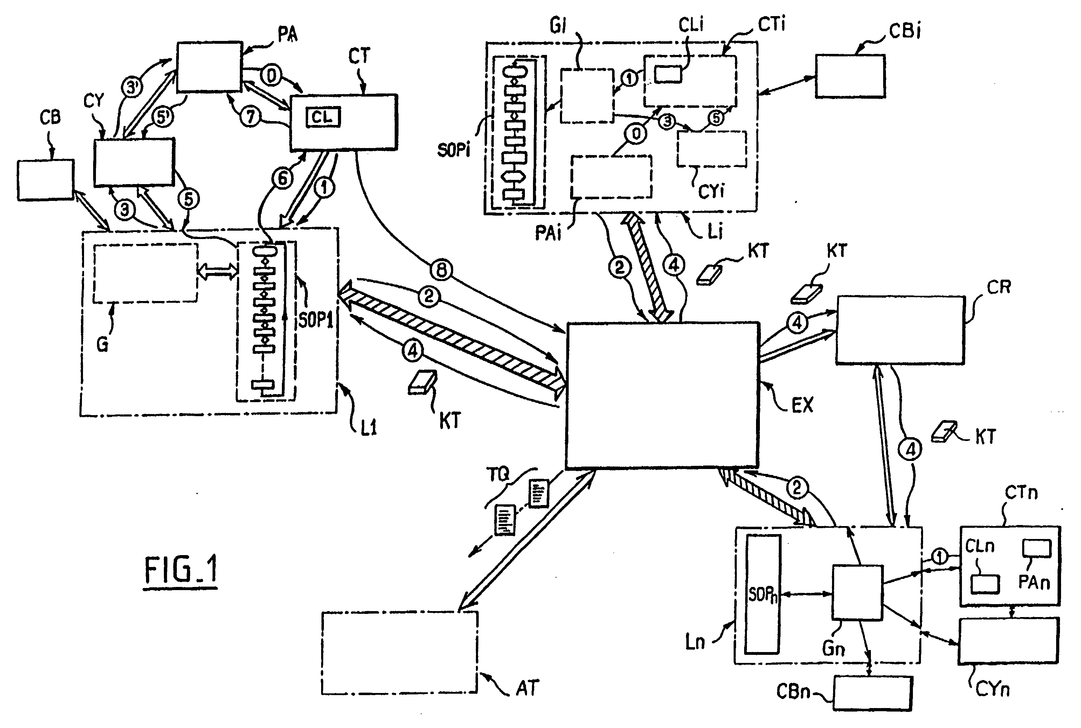

Les parties impliquées dans l'exploitation du procédé selon l'invention sont, en référence à la figure 1:
- une entité EX contrôlant l'exploitation du procédé selon l'invention et diffusant des kits thérapeutiques KT,
- des centres de traitement CT, CTi, CTn animés par des cliniciens CL, CLi, CLn qui, pour leurs patients PA, PAi, PAn, prennent l'initiative de la mise en route de protocoles thérapeutiques,
- des laboratoires de préparation L1, Li, Ln qui préparent, analysent et conditionnent les produits utilisés dans ces protocoles thérapeutiques,
- des services de cytaphérèse CY, CYi, CYn chargés des prélèvements et réinjections sur les patients,

des laboratoires de contrôle bactériologique CB, CBi, CBn, et
éventuellement des centres de retrait CR distincts de l'entité d'exploitation EX.

Les étapes principales du processus thérapeutique contrôlé par le procédé de gestion de qualité selon l'invention sont précisées dans plusieurs exemples illustrés schématiquement en figure 1.

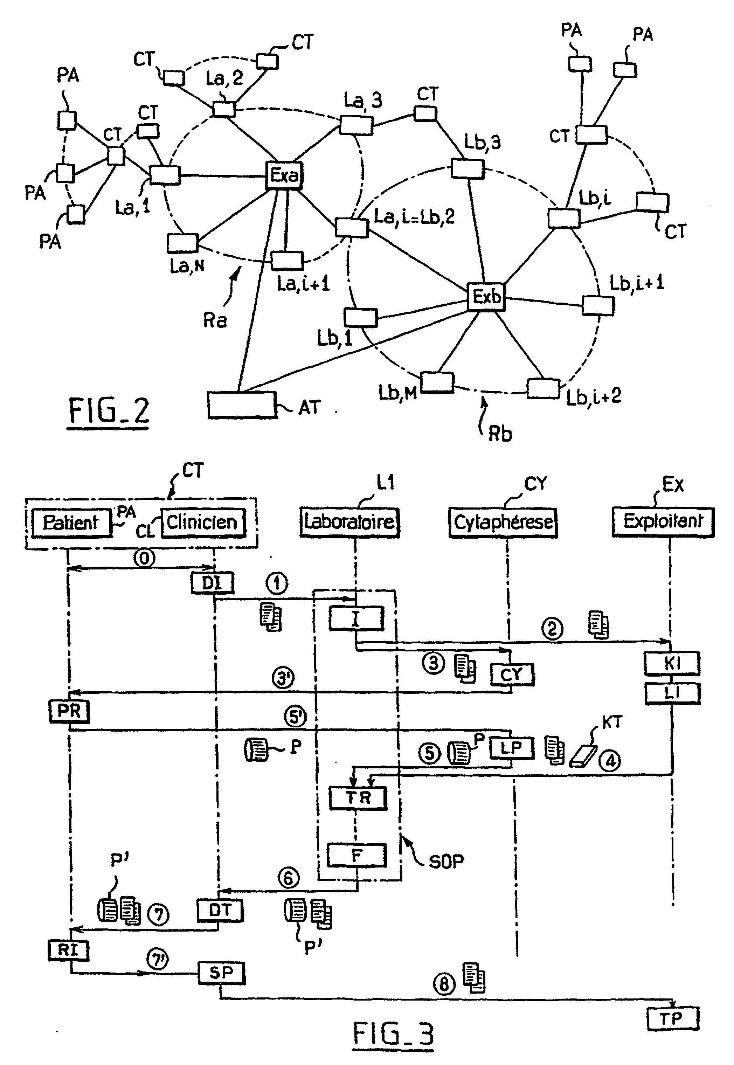

Dans une première configuration rencontrée dans l'exploitation du procédé selon l'invention et décrite séquentiellement en figure 3,
- 1/ un clinicien CL en charge (0) d'un patient PA au sein d'un centre de traitement CT, émet un diagnostic DI et décide de contacter un laboratoire de préparation L1 pour la mise en route d'un ou plusieurs protocoles destinés à son patient,
- 2/ ce laboratoire L1 contacte alors l'entité EX exploitante du protocole thérapeutique procédé pour qu'elle lui fournisse des kits thérapeutiques correspondant à ce protocole,
- 3/ le laboratoire L1 contacte également un service de cytaphérèse CY en vue d'organiser un prélèvement de plasma sur la patient; un prélèvement PR est effectué sur le patient;
- 4/ le laboratoire L1 reçoit de l'entité exploitante EX ou d'un centre de retrait le kit thérapeutique KT et une fiche de cytaphérèse associée; la ou les procédures d'exploitation standard (SOPs) sont alors initiées,
- 5/ le laboratoire L1 reçoit du service de cytaphérèse CY des poches de prélèvement;
- 6/ le contenu de ces poches est traité (TR) par le laboratoire suivant une procédure opératoire standard SOP et sous contrôle du procédé de gestion de qualité selon l'invention,
- 7/ puis, lorsqu'une certification finale F est accordée, les poches traitées accompagnées des documents nécessaires sont transmises au clinicien qui fait réaliser une réinjection RI sur le patient,
- 8/ des informations de suivi post-réinjection sont saisies (7') et communiquées à l'entité exploitante EX.

L'entité exploitante se trouve au coeur du processus thérapeutique et maîtrise le contrôle de la qualité et garantit la traçabilité indispensable pour ce type d'opérations. Le procédé de gestion de qualité est intimement lié à la procédure opératoire SOP1 mise en oeuvre dans le laboratoire L1, mais il peut également être impliqué dans la gestion G de ce laboratoire.

Dans une autre configuration possible d'exploitation du procédé de gestion de qualité selon l'invention, une partie des entités intervenant dans le processus thérapeutique est intégrée sur un seul site. Ainsi, le laboratoire Li en charge de la préparation peut par exemple inclure le centre de traitement CTi et ses cliniciens CLi en charge de patients PAi et des services de cytaphérèse CYi, le contrôle bactériologique étant assuré par un laboratoire extérieur CBi. Le procédé de gestion de qualité selon l'invention peut alors être mis en oeuvre à la fois pour suivre la procédure opératoire SOPi et pour assurer la gestion de qualité Gi au sein des autres entités CYi, CTi.

On peut également prévoir que la distribution des kits thérapeutiques ne soit pas directement assurée par l'entité exploitante Ex mais soit confiée à un centre de retrait CR avec lequel un laboratoire de préparation Ln est en contact pour la fourniture des kits. Le procédé de gestion de qualité selon l'invention assure alors le suivi de la procédure SOPn et la gestion Gn de ce laboratoire de préparation qui est également en contact avec un laboratoire de contrôle bactériologique CBn, un laboratoire de cytaphérèse et un ou plusieurs centres de traitements CTn auxquels sont attachés des cliniciens CLn et leurs patients PAn. L'entité d'exploitation Ex reçoit de chaque laboratoire de préparation des informations relatives à la gestion de qualité des procédures opératoires et au contrôle post-réinjection. Ces données sont traitées, analysées et éventuellement transmises à une autorité de tutelle AT.

Le procédé selon l'invention peut être mis en oeuvre pour la gestion de la qualité de plusieurs protocoles thérapeutiques, comme l'illustre la figure 2.

Plusieurs protocoles thérapeutiques peuvent être exploitée par autant d'entités d'exploitation EXa, EXb contrôlant chacune un réseau Ra, Rb de laboratoires de préparation L_{a,1,} L_{a,2}, L_{a,3}, L_{a,i}, L_{a,i+1}, L_{a,N}; L_{b,1}, L_{b,2}, L_{b,3}, L_{b,i}, L_{b,i+1}, L_{b,i+2}, L_{b,M}. Ces laboratoires effectuent des préparations pour des centres de traitement CT de patients PA et sont tous équipés de logiciels implémentant le procédé de gestion de qualité selon l'invention. Les entités d'exploitation EXa, EXb fournissent les laboratoires en kits thérapeutiques, assurent une supervision des opérations de préparation effectuées par les laboratoires, collectent des données de gestion de qualité et en réfèrent par exemple à une autorité de tutelle AT.

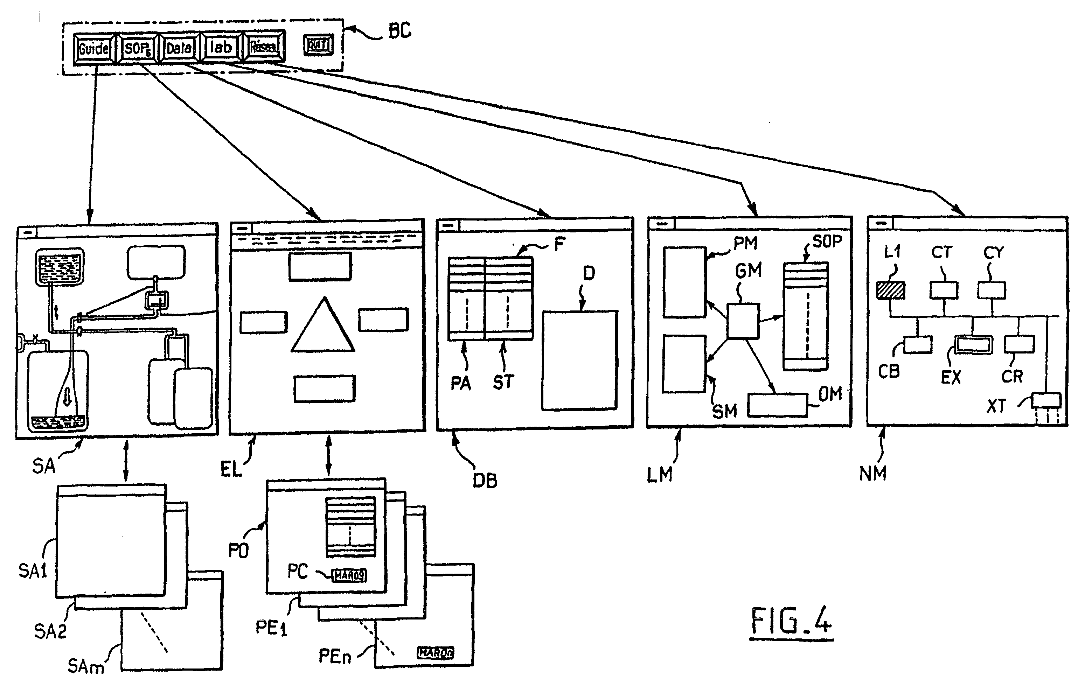

Les outils de traitement d'informations mis en oeuvre avec le procédé de gestion de qualité peuvent en outre assurer d'autres fonctions de contrôle et de traitement, comme l'illustre le schéma de la figure 4. Ainsi, le procédé de gestion de qualité selon l'invention offre à l'opérateur une barre de commandes BC ouvrant notamment accès aux fonctionnalités suivantes:
- une fonctionnalité Guide, qui comprend un ensemble d'informations sur les techniques mises en oeuvre dans le processus thérapeutique, notamment sous la forme de séquences animées SA, SA1, SA2,.., SAm ou de séquences vidéo;
- un suivi complet séquentiel des procédures opératoires standards (SOPs) qui constitue le coeur du logiciel matérialisant le procédé selon l'invention; cet élément logiciel EL comprend notamment un ensemble de pages-écrans PO, PE1,.., PEn qui sont consultées séquentiellement et renseignées par l'opérateur;
- une gestion et un archivage DB des données collectées au cours de l'exploitation du procédé de gestion de qualité;
- une gestion LM du laboratoire de préparation qui peut inclure une gestion des stocks SM, une gestion des personnels PM intégrant notamment des besoins en formation et en évaluation permanente de ces personnels, une gestion OM des entrées-sorties, et une gestion automatisée GM des procédures opératoires standards;
- une fonctionnalité réseau NM pour gérer les communications du laboratoire L1 avec les autres entités intervenant dans les protocoles thérapeutiques, notamment avec des laboratoires de contrôle bactériologique CB, avec des centres de traitement CT, des services de cytaphérèse CY, une entité d'exploitation EX, un centre de retrait CR et une connexion XT à d'autres réseaux.

On va maintenant décrire les étapes principales du procédé de gestion de qualité selon l'invention, en référence aux pages-écrans correspondantes rencontrées par les opérateurs. On considère ici que le procédé de gestion de qualité selon l'invention est mis en ouvre sous la forme d'un logiciel installé sur un poste de travail informatique au sein d'un laboratoire de préparation.

A la mise en route du procédé selon l'invention, un contrôle d'accès est réalisé suivant les techniques connues en ce domaine, par exemple la requête d'un mot de passe et sa vérification; le mot de passe ou code peut être régulièrement validé par des tests d'auto-évaluation des connaissances de l'utilisateur à l'aide d'un programme interactif.

L'opérateur est ensuite sollicité, dans une page-écran d'accueil, pour fournir des données d'identifications des différentes parties impliquées dans le processus thérapeutique auquel est associé le procédé de contrôle selon l'invention.

A titre d'exemple non limitatif, dans le cas du protocole MAK™ de thérapie cellulaire, sont demandées à l'opérateur les informations sur les entités suivantes:
- le clinicien,
- le laboratoire en charge de la préparation du protocole MAK™,
- le laboratoire en charge du contrôle bactériologique,
- le laboratoire en charge de l'aphérèse,
- le centre de traitement.

A chaque demande d'information sur une entité participant au protocole, est associée une page-écran dont les points critiques doivent être intégralement renseignés par l'opérateur pour pouvoir passer à la page-écran suivante, ceci à des fins de sécurité et de traçabilité. Il est à noter que chaque page-écran comporte une identification codée d'un patient.

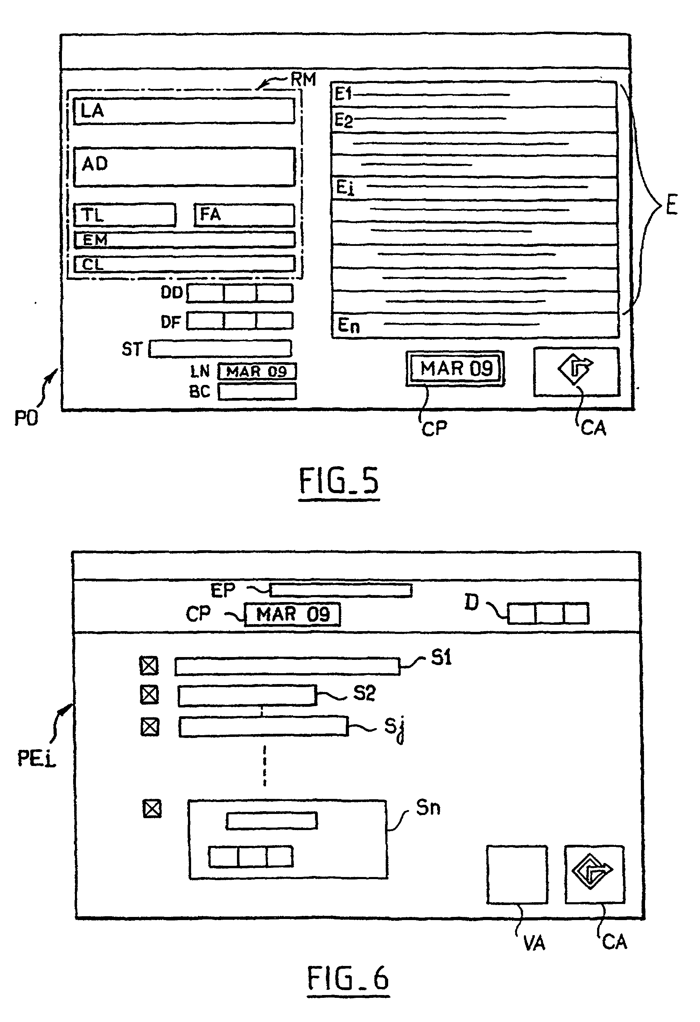

On va maintenant décrire plusieurs exemples de pages-écrans conçues pour chacune des étapes du module SOP du procédé de gestion de qualité selon l'invention, en référence aux figures 5 à 12. N'est présenté ci-dessous qu'un nombre limité de pages-écrans caractéristiques du procédé.

Une page-écran PO (Fig.5) fournit à l'opérateur du procédé une table des matières E, et les coordonnées RM du laboratoire en charge de la préparation, notamment le nom LA de ce laboratoire, son adresse postale AD, ses coordonnées téléphone TL, fax FA et courrier électronique EM ainsi que le nom CL de la personne en charge des préparations. La page-écran PO contient en outre des date DD, DF de début et de fin des préparations, et des champs d'identification essentiels pour le procédé de gestion de qualité selon l'invention;
- un intitulé ST de l'étude,
- un numéro de lot LN,
- un numéro BC de code-barre d'aphérèse,
- un code "patient" CP.

Cette page-écran PO, comme d'ailleurs toutes les pages-écrans développées pour le procédé selon l'invention, utilise un interface graphique et les différentes parties E1, E2,..,Ei,..En du logiciel peuvent être sélectionnées au moyen d'une souris. La page-écran peut être refermée en actionnant une commande CA d'annulation. La saisie des informations peut être faite par clavier, par souris, par la voix ou tout autre moyen de saisie permettant à l'opérateur de travailler dans les conditions opératoires appropriées, notamment d'asepsie.

Après une page-écran (non représentée) correspondant aux paramètres de l'étape de retrait d'un kit de traitement, l'opérateur du procédé de gestion de qualité doit renseigner une page-écran PEi correspondant à la préparation d'un sérum autologue. Cette page-écran comprend, à titre d'exemple non limitatif, un intitulé EP, le rappel du code patient CP et de la date D, et une suite d'instructions opérationnelles S1, S2,.., Sj,..Sn qui doivent être réalisées séquentiellement. A chacune de ces instructions peuvent être associées des données à saisir par l'opérateur. Lorsque l'ensemble de ces opérations a été réalisé, la sélection d'une touche de validation VA commande la fermeture de la page qui n'est confirmée qu'à la condition que toutes les instructions aient été effectuées.

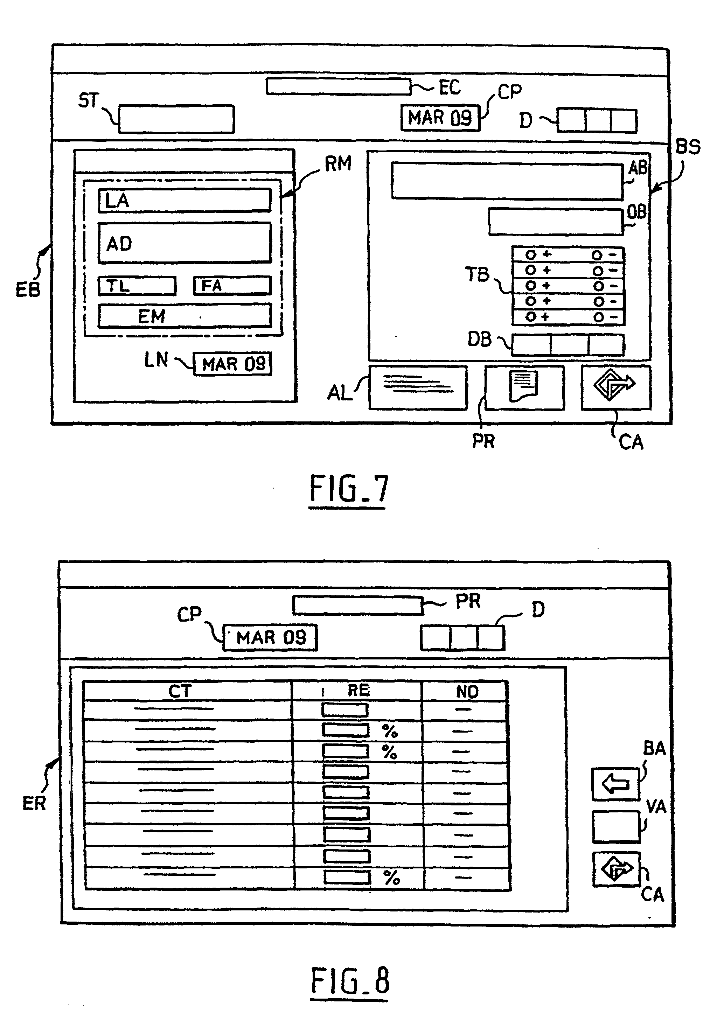

Des étapes de contrôle bactériologique sont prévues tout au long du processus de préparation. La page-écran EB (Fig. 7) correspond à l'une de ces étapes de contrôle bactériologique. Elle comprend généralement un en-tête incluant un intitulé EC, un rappel de l'étude ST, du code patient CP et de la date D, un module RM indiquant les coordonnées du laboratoire responsable de la préparation et le numéro de lot LN, et un module BS rassemblant des données relatives au contrôle bactériologique, notamment l'adresse AB du laboratoire chargé du contrôle bactériologique, l'indication OB d'un opérateur de ce contrôle, les résultats TB d'un ensemble de tests de contrôle bactériologique et la date DB de ces tests. Cette page-écran EB comprend en outre une information AL sur le caractère obligatoire de l'impression de cette page-écran. Un icône de commende d'impression PR est prévu à cet effet.

Lorsque toutes les étapes de la procédure opératoire standard SOP ont été exécutées, une page-écran ER rassemble un ensemble de résultats de tests de qualité qui doivent être saisis pas l'opérateur. Cette page-écran ER, comprend un en-tête semblable comportant un intitulé PR, un rappel du code patient CP et la date d'opération D, et un tableau énumérant une suite de tests quantitatifs CT à chacun desquels est associé un champ "résultat" RE qui doit être renseigné par l'opérateur, et un champ "normes" contenant les valeurs maximales ou minimales constituant les normes. Un icône BA permet à l'opérateur de revenir sur des pages-écrans précédentes.

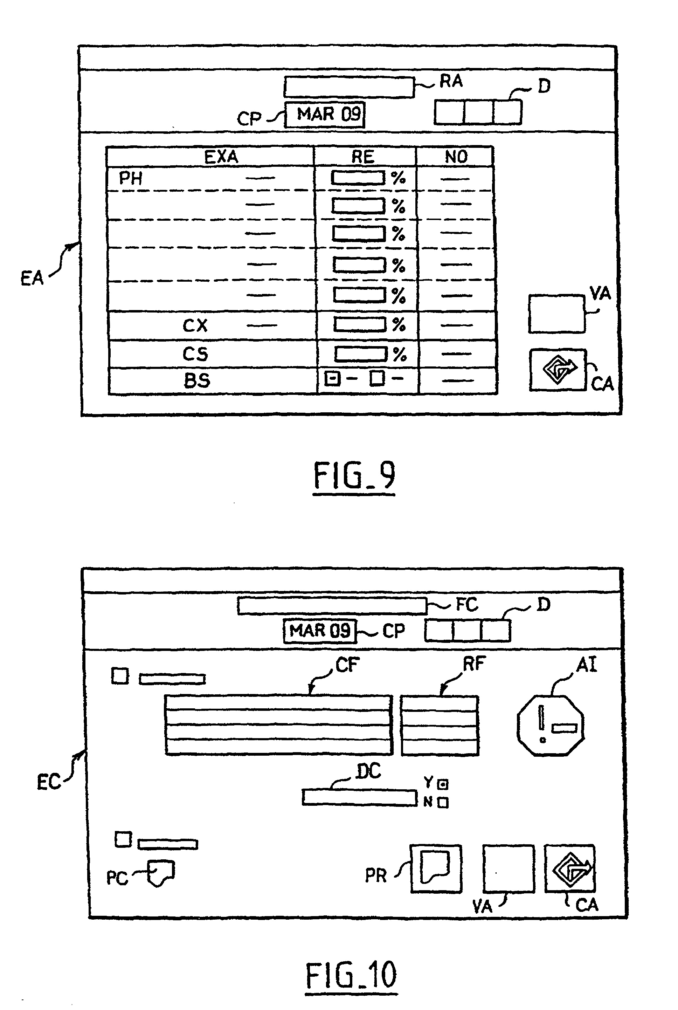

Une page-écran EA (Fig.9) d'analyse rétrospective doit être renseignée par l'opérateur à l'issue de la réinjection des cellules traitées. Cette page-écran EA contient un en-tête incluant un intitulé RA de l'étape, le code patient CP et la date D, et un tableau regroupant, pour un ensemble EXA d'examens PH, CX, CS, BS pratiqués après réinjection, des résultats RE qui doivent être saisis par l'opérateur et, en face de chaque résultat, la norme NO correspondante.

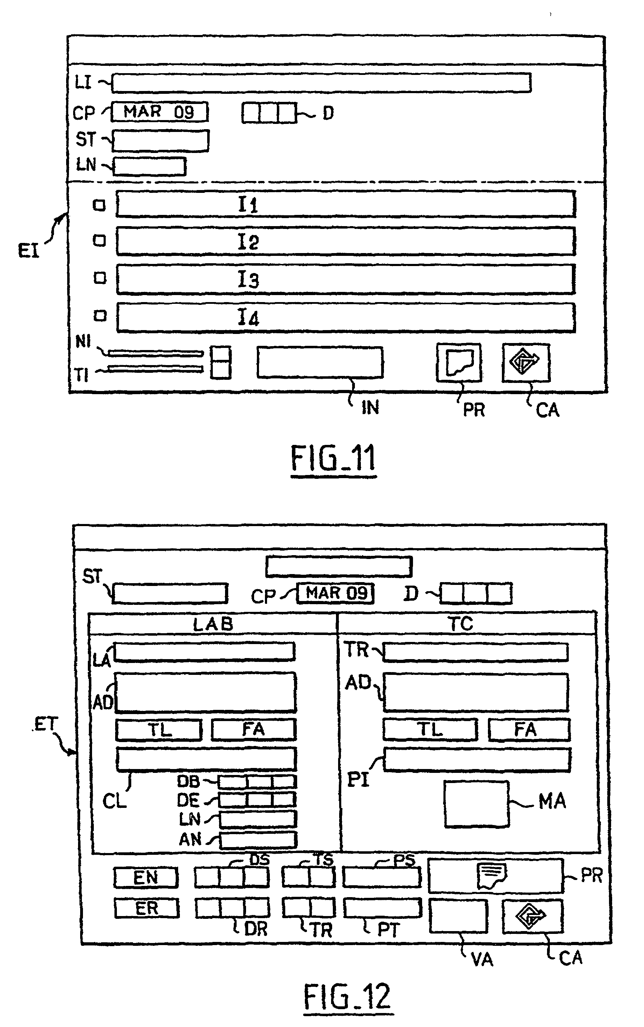

La certification finale d'une préparation est obtenue à partir d'une page-écran spécifique EC (Fig.10) qui, outre l'en-tête d'identification incluant un intitulé FC, le code patient CP et la date D, comporte une déclaration CF de certification finale fournissant des résultats RF quantitatifs et qualitatifs significatifs pour des grandeurs et caractéristiques physiologiques CF: nombre de cellules, viabilité, pourcentage, stérilité. Suit une déclaration de certification finale DC dans laquelle il est indiqué par Oui (Y) ou par Non (N) si la certification finale est effectivement accordée à cette préparation. Un icône d'alarme AI invite l'opérateur à consulter une page-écran EI (Fig.11) listant les irrégularités détectées au cours du processus. Sur la page-écran EC de certification finale, un icône spécifique PC permet à l'opérateur de passer à la page-écran ET (Fig.12) de transfert de la préparation vers le centre de traitement. Par ailleurs, il est normalement prévu une commande d'impression PR, une commande de validation VA et une commande d'annulation CA. Il est à noter que cette page-écran de certification EC ne peut être validée qu'après saisie et vérification d'un mot de passe, pour des raisons de sécurité de la certification.

La page-écran EI d'irrégularités comporte un en-tête incluant un intitulé LI, le rappel du code patient CP, de la date D, de l'étude ST et du numéro du lot LN. Suit une liste I1-I4 d'irrégularités détectées au cours du processus avec mention des remèdes qui y ont été apportés. Cette page-écran EI comporte en outre une indication NI du nombre d'irrégularités vérifiées et une indication TI du nombre total d'irrégularités. Un message IN invité l'opérateur à consulter la page-écran correspondant à une irrégularité donnée en effectuant un double-clic à l'aide de la souris sur l'irrégularité visée.

Après consultation éventuelle de cette page-écran d'irrégularités, l'opérateur peut alors consulter et traiter la page-écran ET de transfert vers le centre de traitement. Cette page-écran comporte, outre un en-tête incluant un intitulé, le rappel du code patient CP, de l'étude ST et de la date D, d'une part un ensemble de données LAB caractéristiques du laboratoire responsable de la préparation, et d'autre part les coordonnées TC du centre de traitement auquel est destinée la préparation.

Les caractéristiques LAB incluent pour le laboratoire, le nom LA, l'adresse postale AD, les coordonnées téléphoniques et fax TL, FA, le nom CL de la personne en charge de la préparation. Les informations TC relatives au centre de traitement incluent son intitulé TR, son adresse AD, ses numéros de téléphone et de fax TL, FA et le nom PI de la personne en charge de ce traitement.

Un message MA alerte l'opérateur sur l'obligation d'imprimer cette page-écran, qui comprend en outre, par exemple en bas de page, des informations relatives aux opérations d'envoi et de réception. Ainsi, les lignes d'envoi EN et de réception ER doivent être renseignées par l'opérateur pour les champs suivantes: dates DS, DR; heures TS, TR et personnes responsables PS, PT.

Bien sûr, l'invention n'est pas limitée aux exemples qui viennent d'être décrits et de nombreux aménagements peuvent être apportés à ces exemples sans sortir du cadre de l'invention. Ainsi, ce procédé peut être appliqué au contrôle et au suivi de qualité dans bien d'autres domaines que celui de la thérapie cellulaire. Il peut en outre être intégré dans des procédé d'automatisation de laboratoire. Le procédé selon l'invention s'inscrit également dans les préoccupations actuelles de biovigilance et de pharmacovigilance.

## Revendications

1. Procédé de traitement d'informations utilisé pour la gestion de qualité dans un processus thérapeutique, ce processus thérapeutique comportant des opérations de prélèvement de cellules (PR) sur un patient (PA), des opérations de traitement spécifique de ces cellules selon un protocole de traitement spécifique, et une opération de réinjection sur ce patient desdites cellules ainsi traitées, ces opérations de prélèvement, de traitement et de réinjection étant soumises à une procédure opératoire standard de préparation (SOP) comportant une succession d'étapes fonctionnelles,
**caractérisé en ce qu'**il comprend, pour chaque lot de prélèvement effectué sur un patient donné :
- pour chaque étape fonctionnelle, une étape de validation (VA) séquentielle et conditionnelle de ladite étape, le passage d'une. étape de validation à l'étape de validation suivante étant conditionné par des résultats d'un traitement de données collectées au cours de cette étape de validation, et
- une étape de traitement des informations et données collectées dans les différentes étapes de validation, ces données collectées étant associées audit lot de prélèvement et étant indicatives notamment des intervenants et de - l'état d'avancement du processus, pour émettre une certification finale (CF) d'une préparation réalisée selon la procédure opératoire standard et/ou une liste des irrégularités détectées au cours de cette préparation.

2. Procédé selon la revendication 1, **caractérisé en ce que** la validation de la certification finale est conditionnée à l'entrée d'un mot de passe de validation.

3. Procédé selon l'une des revendications 1 ou 2, mis en oeuvre dans un système de traitement d'informations, **caractérisé en ce que** chaque étape de validation est associée au moins une page-écran (PO, Pei, EP, EA, EC, EI) accessible sur des moyens de visualisation d'au moins un poste de travail connecté au système de traitement d'informations.

4. Procédé selon la revendication 3, caractérisé en ce chaque page-écran comprend un champ d'identification codée d'un patient auquel correspond le lot de prélèvement soumis à la procédure opératoire standard.

5. Procédé selon l'une des quelconques revendications précédentes, **caractérisé en ce que** la sortie de certaines des étapes (RA) dudit procédé est conditionnée à l'impression de pages-écran (EA) correspondant à ces étapes.

6. Système de traitement d'informations utilisé pour la gestion de qualité dans un processus thérapeutique, ce processus thérapeutique comportant des. opérations de prélèvement de cellules (PR) sur un patient (PA), des opérations de traitement spécifique de ces cellules selon un protocole de traitement spécifique (SOP), et une opération de réinjection (RI) sur ce patient desdites cellules ainsi traitées, ces opérations de prélèvement, de traitement et de réinjection étant soumises à une procédure opératoire standard de préparation comportant une succession d'étapes fonctionnelles (TR),
**caractérisé en ce qu'**il comprend, pour chaque lot de prélèvement effectué sur un patient donné:
- pour chaque étape fonctionnelle, un moyen de validation (VA) séquentielle et conditionnelle de ladite étape, le passage d'une étape de validation à l'étape de validation suivante étant conditionné par des résultats d'un traitement de données collectées au cours de cette étape de validation, et
- un moyen de traitement des informations et données collectées dans les différentes étapes de validation, ces données collectées étant associées audit lot de prélèvement et étant indicatives notamment des intervenants et de l'état d'avancement du processus, pour émettre une certification finale (CF) d'une préparation réalisée selon la procédure. opératoire standard et/ou une liste des irrégularités détectées au cours de cette préparation.

7. Système selon la revendication 6, mis en oeuvre dans un laboratoire de préparation, **caractérisé en ce qu'**il est en outre agencé pour exécuter des tâches de gestion (GN) au sein de ce laboratoire.

8. Système selon la revendication 7, **caractérisé en ce qu'**il est connecté à un réseau de communication pour échanger des données avec d'autres entités (CTn, Ln, Cyn, CRn, CB) impliquées dans un processus thérapeutique.

9. Application du procédé et du système de traitement d'informations utilisés pour la gestion de qualité selon l'une guelconque des revendications précédentes, à des protocoles de thérapie cellulaire.

10. Application du procédé et du système de traitement d'informations utilisés pour la gestion de qualité selon l'une quelconque des revendications précédentes, à des protocoles de thérapie génique.

11. Application du procédé et du système de gestion de qualité selon l'une quelconque des revendications précédentes, permettant la formation continue de l'opérateur et/ou le contrôle de son niveau de connaissance.

## Patentansprüche

1. Informations-Verarbeitungsverfahren, welches zum Qualitätsmanagement in einem therapeutischen Prozess benutzt wird, wobei dieser therapeutische Prozess Zellentnahme-Vorgänge (PR) an einem Patienten (PA), spezifische Behandlungsvorgänge an diesen Zellen nach einem spezifischen Behandlungsprotokoll und einen Wiederinjektions-Vorgang an diesem Patienten mit den so behandelten besagten Zellen aufweist, und wobei diese Entnahme-, Behandlungs- und Wiederinjektions-Vorgänge einer eine Folge von funktionellen Schritten aufweisenden Präparierungs-Standard-Arbeitsanweisung (SOP) unterworfen sind,
**dadurch gekennzeichnet, daß**
das Verfahren für jede an einem gegebenen Patienten entnommene Probe folgendes aufweist:
- für jeden funktionellen Schritt einen für den besagten Schritt sequentiellen und konditionellen Validierungs-Schritt (VA), wobei der Übergang von einem Validierungs-Schritt auf den nächsten von den Ergebnissen der Verarbeitung der im Laufe dieses Validierungs-Schritts gesammelten Daten abhängt, und
- einen Verarbeitungs-Schritt der während der verschiedenen Validierungs-schritte gesammelten Informationen und Daten, wobei die gesammelten Daten der besagten entnommenen Probe zugeordnet und insbesondere kennzeichnend für die Beteiligten und den Fortschritt des Prozesses sind, um eine endgültige Zertifizierung (CF) einer Präparierung entsprechend der Standard-Arbeitsanweisung und/oder eine Liste von während dieser Präparierung entdeckter Unregelmäßigkeiten ausgeben zu können.

2. Verfahren nach Anspruch 1,
**dadurch gekennzeichnet, daß**
die Validierung der endgültigen Zertifizierung von der Eingabe eines Validierungs-Passwortes abhängt.

3. Verfahren nach einem der Ansprüche 1 oder 2, durchgeführt in einem Informations-Verarbeitungssystem,
**dadurch gekennzeichnet, daß**
jeder Validierungs-Schritt mindestens einer Bildschirmseite (PO, Pei, EP, EA, EC, EI) zugeordnet ist , welche auf Visualisierungsmitteln mindestens eines an ein Informations-Verarbeitungssystem angeschlossenen Arbeitsplatzes zugänglich ist.

4. Verfahren nach Anspruch 3, **dadurch gekennzeichnet, daß** jede Bildschirmseite ein kodiertes Identifizierungsfeld eines Patienten, dem die einer Standard-Arbeitsanweisung unterworfene entnommene Probe zugeordnet ist, aufweist.

5. Verfahren nach irgendeinem der vorangehenden Ansprüche, **dadurch gekennzeichnet, daß**
die Ausgabe gewisser Schritte (RA) des besagten Verfahrens vom Ausdrucken der diesen Schritten entsprechenden Bildschirmseiten (EA) abhängt.

6. Informations-Verarbeitungssystem, welches zum Qualitätsmanagement in einem therapeutischen Prozess benutzt wird, wobei dieser therapeutische Prozess Zellentnahme-Vorgänge (PR) an einem Patienten (PA), spezifische Behandlungsvorgänge an diesen Zellen nach einem spezifischen Behandlungsprotokoll (SOP) und einen Wiederinjektions-Vorgang (RI) an diesem Patienten mit den so behandelten besagten Zellen aufweist und wobei diese Entnahme-, Behandlungs- und Wiederinjektionsvorgänge einer eine Folge von funktionelle Schritten (TR) aufweisenden Präparierungs-Standard-Arbeitsanweisung (SOP) unterworfen sind,
**dadurch gekennzeichnet, daß**
das Verfahren für jede an einem gegebenen Patienten entnommene Probe folgendes aufweist:
- für jeden funktionellen Schritt ein für den besagten Schritt sequentielles und konditionelles Validierungs-Mittel (VA), wobei der Übergang von einem validierungs-Schritt auf den nächsten von den Ergebnissen der Verarbeitung der im Laufe dieses Validierungs-Schritts gesammelten Daten abhängt, und
- ein Verarbeitungs-Mittel zur Verarbeitung der während der verschiedenen Validierungs-Schritte gesammelten Informationen und Daten, wobei die gesammelten Daten der besagten entnommenen Probe zugeordnet und insbesondere kennzeichnend für die Beteiligten und den Fortschritt des Prozesses sind, um eine endgültige Zertifizierung (CF) einer Präparierung entsprechend der Standard-Arbeitsanweisung und/oder eine Liste von während dieser Präparierung entdeckter Unregelmäßigkeiten ausgeben zu können.

7. System nach Anspruch 6, eingesetzt in einem Präparierungs-Labor, **dadurch gekennzeichnet, daß**
es außerdem so ausgeführt ist, daß es Verwaltungsaufgaben (GN) in diesem Labor durchführen kann.

8. System nach Anspruch 7, **dadurch gekennzeichnet, daß**
es an ein Kommunikationsnetz für den Datenaustausch mit anderen an diesem therapeutischen Prozess beteiligten Einheiten ( CTn, Ln, Cyn, CRn, CB) angeschlossen ist.

9. Anwendung des für das Qualitätsmanagement benutzten Verfahrens und Systems zur Informations-Verarbeitung nach irgendeinem der vorangehenden Ansprüche auf Zelltherapieprotokolle.

10. Anwendung des für das Qualitätsmanagement benutzten Verfahrens und Systems zur Informations-Verarbeitung nach irgendeinem der vorangehenden Ansprüche auf Gentherapieprotokolle.

11. Anwendung des für das Qualitätsmanagement benutzten Verfahrens und Systems zur Informations-Verarbeitung nach irgendeinem der vorangehenden Ansprüche, wodurch die berufliche Fortbildung der Bedienungsperson und/oder die Kontrolle ihres Wissensstandes ermöglicht werden.

## Claims

1. Process for the processing of information used for the management of quality in a therapeutic process, this therapeutic process comprising the operations of taking cells (PR) from a patient (PA), specific treatment operations on these cells using a specific treatment protocol, and a reinjection operation into the patient of said cells treated in this way, these operations of taking cells, treatment and reinjection being subjected to a standard operating procedure for preparation (SOP) comprising a series of functional stages,
**characterized in that** it comprises, for each batch of samples taken from a given patient:
- for each functional stage, a stage of sequential and conditional validation (VA) of said stage, the passing from one validation stage to the following validation stage being conditional on the results of the processing of data collected during this validation stage, and
- a stage of processing of the information and data collected in the different validation stages, these collected data being associated with said batch of samples and being indicative particularly of the involved parties and of the progress of the process, in order to issue final certification (CF) of a preparation carried out according to the standard operating procedure and/or a list of the anomalies detected during this preparation.

2. Process according to claim 1, **characterized in that** validation of the final certification is conditional on the input of a validation password.

3. Process according to one claims 1 or 2, implemented in a data processing system, **characterized in that** with each validation stage is associated at least one screen page (PO, Pei, EP, EA, EC, EI) which can be accessed on the display means of at least one workstation connected to said data processing system.

4. Process according to claim 3, **characterized in that** each screen page comprises a coded identification field for a patient which matches the batch of samples subjected to the standard operating procedure.

5. Process according to any one of the previous claims,
**characterized in that** the exit from certain stages (RA) of said process is conditional on printing the screen pages (EA) corresponding to these stages.

6. System for the processing of information used for the management of quality in a therapeutic process, this therapeutic process comprising the operations of taking cells (PR) from a patient (PA), specific treatment operations on these cells using a specific treatment protocol (SOP), and a reinjection operation (RI) into the patient of said cells treated in this way, these operations of taking cells, treatment and reinjection being subjected to a standard operating procedure for preparation comprising a series of functional stages (TR),
**characterized in that** it comprises, for each batch of samples taken from a given patient:
- for each functional stage, a means of sequential and conditional validation (VA) of said stage, the passing from one validation stage to the following validation stage being conditional on the results of the processing of data collected during this validation stage, and
- a means of processing of the information and data collected in the different validation stages, these collected data being associated with said batch of samples and being indicative particularly of the involved parties and of the progress of the process, in order to issue final certification (CF) of a preparation carried out according to the standard operating procedure and/or a list of the anomalies detected during the preparation.

7. System according to claim 6, implemented in a preparation laboratory, **characterized in that** it is further designed to execute management tasks (GN) within this laboratory.

8. System according to claim 7, **characterized in that** it is connected to a communications network in order to exchange data with other entities (CTn, Ln, Cyn, CRn, CB) involved in a therapeutic process.

9. Application of the process and of the information processing system used for quality management according to any one of the previous claims to cell therapy protocols.

10. Application of the process and of the information processing system used for quality management according to any one of the previous claims to gene therapy protocols.

11. Application of the process and of the quality management system according to any one of the previous claims, allowing ongoing training of the operator and/or the monitoring of his or her level of knowledge.
